# EUROPEAN PATENT APPLICATION

(11) **EP 2 316 528 A1**
(43) Date of publication of application: **04.05.2011**
(21) Application number: 09425435.6
(22) Date of filing: 30.10.2009
(51) Int. Cl.: A61N 5/10, G21K 1/00

(54) **Radiological treatment laser device and related surgical apparatus**

(71) Applicant: Consiglio Nazionale Delle Ricerche, 00185 Roma (IT)
(72) Inventor: Giulietti, Antonio, 56126 Pisa (IT); Gamucci, Andrea, 56028 San Miniato (PI) (IT); Gizzi, Leonida Antonio, 56125 Pisa (IT)
(74) Representative: Leone, Mario

(57) **Abstract**

An innovative device is proposed for the radiological treatment of tumours, in particular per irradiating a surgical site according to the so-called IORT (IntraOperative Radiological Therapy) technique. It operates with a not-high vacuum, and it allows to physically placing the laser source away from the focusing means, thus allowing to provide a small sized, easily handlable device that can be used in the operating room and easily substituted for maintenance, therefore without interfering with the use of the room itself. The apparatus provides a relativistic electron generator having a kinetic energy and average current suitable for IORT and having: a laser source (2) for generating a laser beam (5); a nozzle (10) for vacuum emitting a supersonic gas jet (50); focusing means for focusing (11) said laser beam (5) on said pressurized gas jet (50) nearby the nozzle (10); and adjustment means for adjusting the electrons kinetic energy according to the respective therapeutic needs by means of varying the gas jet (50) thickness (b). The accelerating device is miniaturized and operates in high efficiency laser acceleration conditions experimentally determined by the inventor.

## Description

The present invention refers to a radiological treatment device, e.g. of the type used for irradiating a surgical site in particular according to the so-called IORT (Intra-Operative Radiological Therapy) technique, for tumours treatment.

According to this technique, the patient is operated for the extirpation of tumoral masses, after which, before closing the surgical site, it is treated with radiations avoiding or delaying the growth of further tumoral masses. To this scope, the radiation is directed through the wound directly opened on the site, thus avoiding passing through the corporal mass. This technique differs from other irradiating techniques (radiotherapy), wherein the tumoral site is radiologically treated through the corporal mass.

The passage through the corporal mass can cause damages to the tissues, in particular for the relevant amount of energy required for assuring the penetration of the radiation, which should reach the tumoral site with a sufficient energy for the reduction thereof. A radiological device operating externally the corporal mass is for example described in the US patent application No. 2008/0298401 A1.

The IORT technique generally requires the use of electrons. Known devices generates an electron beam by means of a linear accelerator, i.e. a resonant cavity which is resonant to the radio-frequency generated by a high power radio-frequency generator requiring Ultra-High Vacuum (UHV). A moderately relativistic (1-12 MeV) beam is generated, allowing to penetrate tissues typically for few centimetres, although the treatment might require a penetration up to 15 cm, corresponding to an energy of about 25 MeV. A suitable energy amount of the order of 10 Gy is released in about one minute in case of currents of the order of circa 10nA.

Nevertheless, known devices for this technique are bulky and heavy, and they should be placed such that they stand above the patient in the operating room. Due to dimensions and weight, as well as to the requirement of UHV, the use of such device is difficult and requires specialized technical staff.

Moreover, these devices can be hardly moved from the operating room, thereby all maintenance should be executed in loco, with consequent reflection on the aseptic conditions required for the operating room. Moreover, during maintenance, the room cannot be used for other operations.

Furthermore, frequent failures and malfunctions occur both in maintaining the Ultra-High Vacuum conditions and in the radio-frequencies generation, which can at least partially compromise the operation outcome, if they occur during the execution thereof.

Accordingly, the need of a device capable of emitting radiations suitable for the above mentioned purpose, being small sized, easy to handle and to easily removable, is remarkably felt.

The solution concept it based on the use of laser-operated linear electron accelerators, shortly called laser accelerators. This principle has been described for the first time in T. Tajima and J.M. Dawson, Laser Electron Accelerator on Physical Review Letters, Vol. 43, No. 4, 23 July 1979. An accelerating device is also described in US Patent No. 5,789,876 (Umstadter et al.).

The operating principle, briefly described, consists in focusing a laser beam on a pressurized gas jet. The laser incidence turns gas into plasma, and at the same time produces an electron beam having a relativistic velocity, emitted according to the laser propagation axis.

The laser technique, in comparison to the radiofrequency one, can produce electrons of some tens of MeV in few millimetres, whilst, on the contrary, the RF technique requires more than one meter.

The technical problem underlying the present invention is to provide a radiological treatment device as specified above, comprising a electron generator having:
- a laser source for generating a laser beam;
- a nozzle emitting a pressurized gas jet in vacuum; and
- focusing means for focusing said laser beam on said pressurized gas jet nearby the nozzle,
**characterized in that** it comprises adjustment means for adjusting the thickness of the gas jet passed through by the laser.

The main advantage of the above mentioned device consists in that it allows the use of not excessively high-vacuum, and in the possibility of physically placing the laser source away from the focusing means, thus allowing to provide a small sized, easily handlable device that can be used in the operating room and easily substituted for maintenance, therefore without interfering with the use of the room itself.

It should be noted that it is possible to control the kinetic energy of the emitted electrons by adjusting the thickness of the gas jet crossed by the laser.

According to a preferred exemplificative embodiment of the invention, said adjustment means operates by varying the laser propagation axis distance from the gas jet output plane, i.e. the distance from the nozzle output section, so as to exploit the gas jet natural divergence.

Moreover, the adjustment means can operate by varying the laser propagation axis tilt with respect to the nozzle axis, or vice versa. Furthermore, the two effects hereby described can be combined in said adjustment means for adjusting the thickness of the gas jet crossed by the laser propagation axis.

Furthermore, also the gas jet sizes can be varied, e.g. by operating on the nozzle output section sizes and/or providing a set of interchangeable nozzles having different cross-section and an automatic device for inserting the nozzle having the required cross-section.

The nozzle substitution could be used for a coarse adjustment of the crossed distance, while variation of the laser axis distance from the nozzle output and/or the variation of the tilt can be used for a fine adjustment.

The present invention also relates to a surgical apparatus comprising a device as previously described, wherein the laser source is placed in a room separated from the operating room, the laser beam being guided by means of a vacuum tubular pipe to a device body housing the nozzle for vacuum emitting a pressurized gas jet, the focusing means for focusing the laser beam and the adjustment means for adjusting the gas jet thickness crossed by the laser beam.

The present invention will be hereby described according to a preferred exemplificative embodiment thereof, given by way of a non-limiting example, with reference to the annexed figures, wherein:
- figure 1 shows a functional chart describing a surgical apparatus comprising a device for the radiological treatment according to the invention;
- the figure 2 shows the functional chart of figure 1 in order to describe the apparatus logistic;
- figure 3 schematically shows a functional detail of the apparatus shown in the previous figures;
- figure 4 shows a modification of the apparatus of figure 1, which is arranged according to a multiple logic;
- figure 5 shows a functional chart of a part of the apparatus of figure 1, in particular the laser source;
- figure 6 shows a functional chart of a further part of the apparatus of figure 1, in particular the device according to the invention;
- figure 7 shows schematically a further part of the apparatus of figure 1, in particular the detail of figure 3;
- figure 8 shows a functional chart of a further part of the apparatus of figure 1;
- figure 9 shows the operating principle of the relativistic electrons laser generation;
- figure 10 shows a axonometric and schematic view of a component of the device according to the present invention;
- figure 11 pictorially shows an operating room housing a surgical apparatus according to the present invention; and
- figure 12 shows a diagram showing a single electron kinetic energy trend in function of the gas jet thickness crossed by the laser beam.

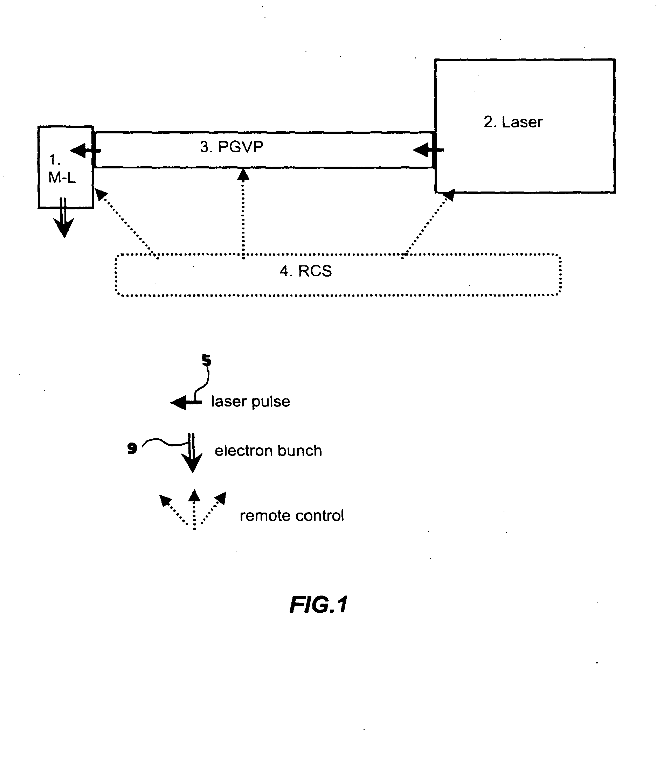

With reference in particular to figure 1, a surgical apparatus including a laser accelerator for generating electrons, to be used according to so-called technique known as IORT, is schematically represented.

In this chart, it is possible to conceptually distinguish an accelerating head 1 housing a miniaturized linear accelerator, in short mini-linac (M-L), and a laser source 2, that will be both described in detail in the following.

The accelerating head 1 and the laser source 2 are connected to a laser pulse guiding pipe 3 in particular a *"pulse guiding vacuum pipe"* (PGVP), wherein vacuum has been created and that will be also described in the following.

These components are controlled by means of a remote control system 4 (RCS) connected thereto and provided with suitable software and with components such as a processor, sensors, actuators and all the required electronics.

The linear accelerator provides an electron beam having the energy required for the IORT technique, with an energy variable from 1 to 25 MeV, with currents of the order of some nA. The accelerator is operated by laser pulses provided by the source 2 and guided by means of the guiding pipe 3.

The latter guides the laser pulses under conditions of ordinary vacuum, with a pressure lower than 0.133 Pa (10⁻³ Torr), preferably lower than 0.013 Pa (10⁻⁴ Torr). Vacuum pressures lower than 0.001 Pa (10⁻⁵ Torr) are not required.

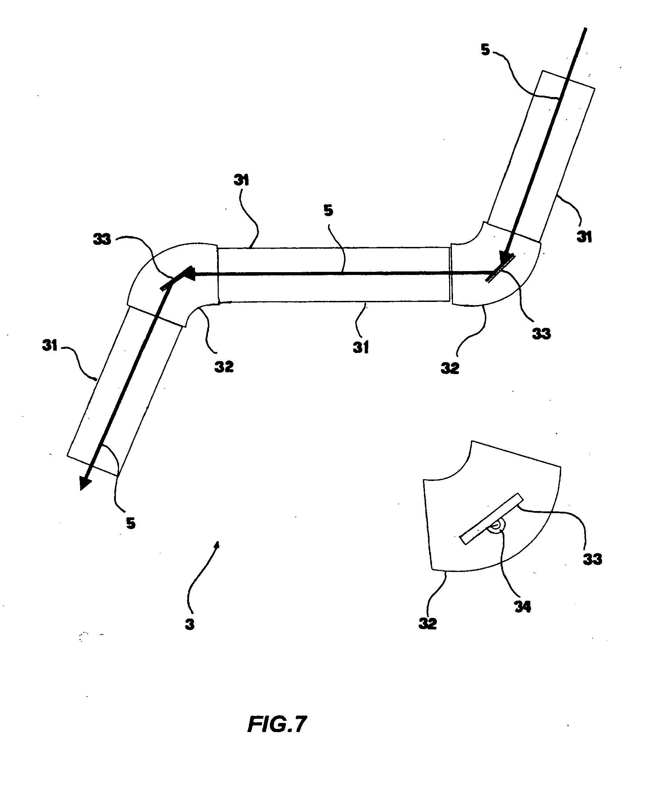

Pulses transmission takes place with the precision required by circumstances and, to this regard, the guiding pipe 3 comprises a plurality of rectilinear tubular elements 31, connected by articulated joints 32 containing an optical element, in particular a mirror 33 mounted on an adjustable support 34 operated by said remote control 4. The mirrors can be plane (figure 7).

In this way, the laser beam, represented by the referral number 5, can follow a complex path, allowing to move away and physically separate the source 2 from laser accelerator (M-L).

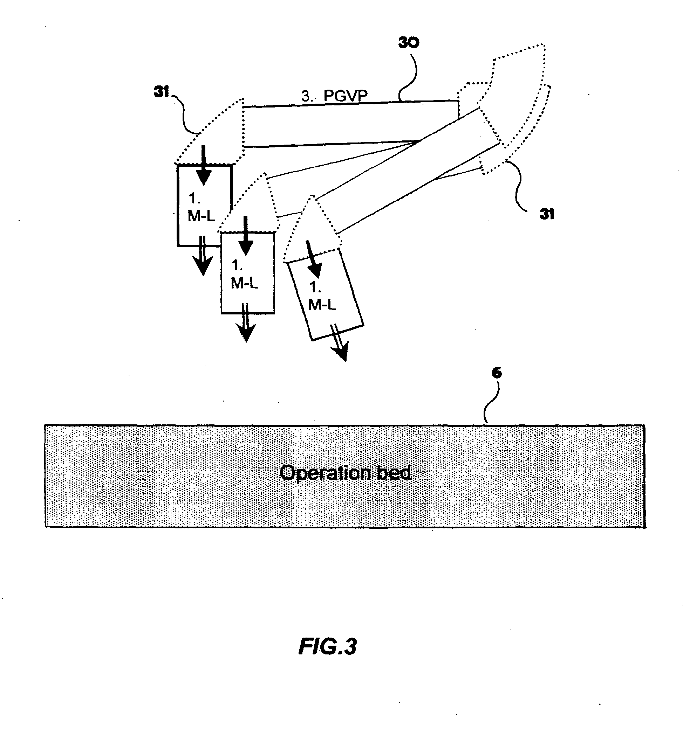

Moreover (figure 3), the articulated joints allows, within the operating room, to move closer and away the radiological treatment device and to adjust the position thereof, i.e. of the head 1 housing the laser accelerator (M-L) relative to the operating bed 6.

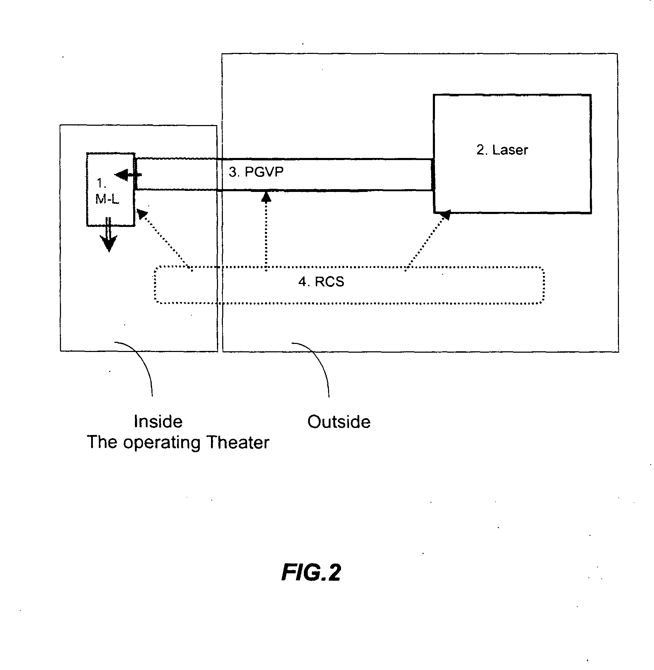

With reference to figure 2, it is shown the arrangement of the surgical apparatus components hereby described.

The laser source 2 is placed in a apposite service room 7, distinct from the operating room 8, and the guiding pipe 3 transmit laser pulses from the service room 7 into the room 8, i.e. to the laser accelerator.

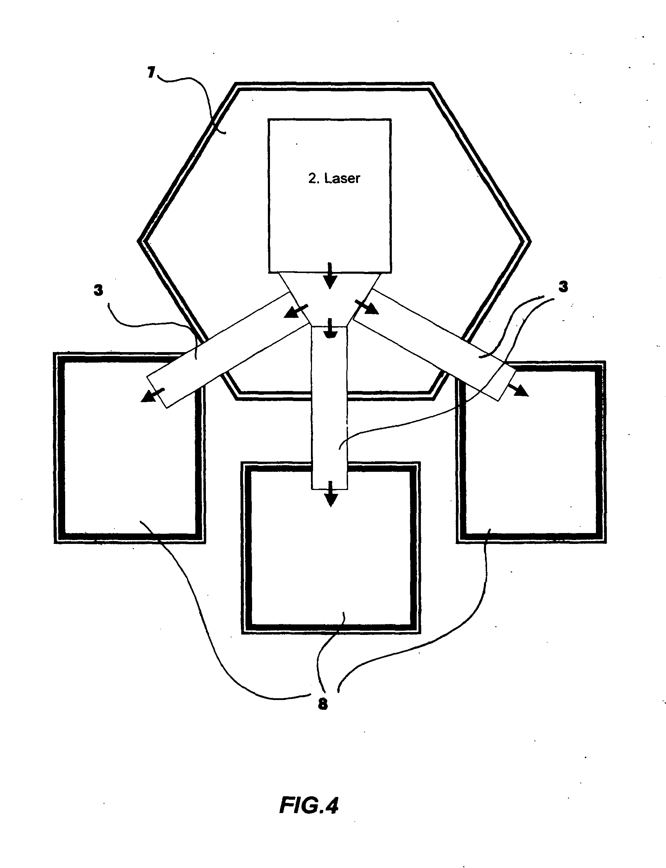

According to a different arrangement shown in figure 4, a single service room 7 housing one or more laser sources can serve any number of a operating rooms 8 by means of respective guiding pipes 3, realized as described with reference to figures 3 and 7.

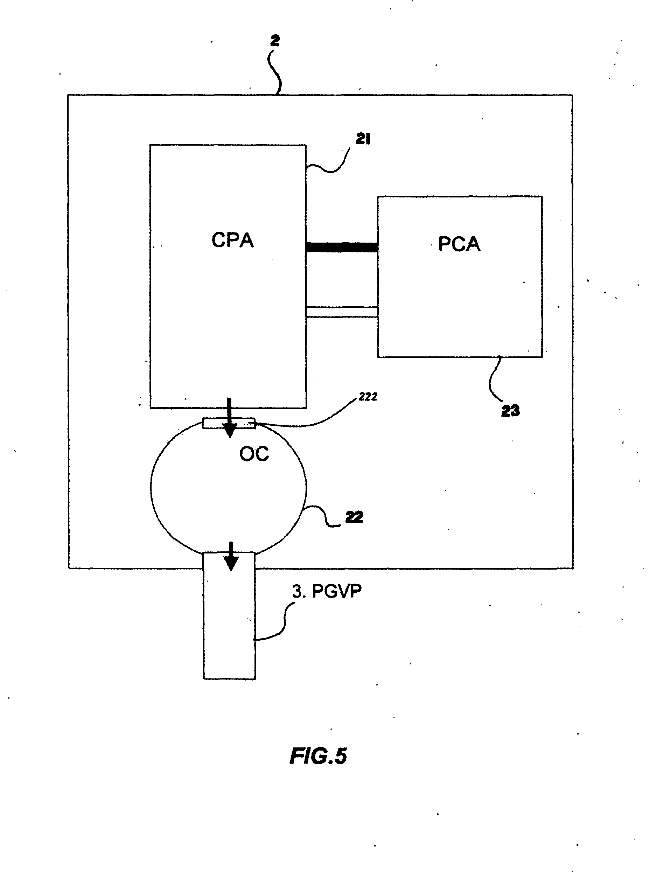

Laser source 2 (figure 5) is of Ti:Sapphire type based on CPA (chirped pulse amplification) technique. CPA laser system 21 comprises an oscillator with a repetition frequency comprised between 50 and 100 MHz, preferably between 70 and 90 MHz, emitting pulse trains of duration comprised between 10 and 30 fs, preferably between 15 and 25 fs, with a wavelength equal to about 800 nm, a bandwidth of the order of 40 nm and an energy of the order of nJ.

A single pulse is extracted by means of a Pokels cell, the pulse being sent to a *time stretcher* (formed by an optical fibre or two diffraction gratings) stretching the pulse until a duration of 300+900 ps.

This pulse is then three stage-amplified by means of a series of optical amplifiers, normally having three or four stages, until an energy comprised between 0.5 and 2.0 J is reached. The pulse final optical compression provided by the two gratings within the optical compressor 22 (OC) provides a pulse duration comprised between 20 and 500 fs, preferably between 50 and 200 fs. As the OC should operate under vacuum conditions, the laser beam is inputted by means of the vacuum sealed optical window 222.

Within these ranges, an acceleration state with ultra-short pulses with duration lower than 50 fs, and a short pulses state with duration higher than 50 fs, i.e. comprised between 50 fs and 200 fs in the preferred example, are defined.

The two states differ in the physical features of the acceleration process and in the electron production efficiency (i.e. the ratio between the total energy contained in the relativistic electrons pack and the laser pulse energy). The first state is closer to the ideal condition described by the theory of the Laser-Wake-Field-Acceleration (LWFA) process, a theory first presented in the above mentioned Tajima and Dawson work. Such conditions requires a sort of resonance between pulse duration and plasma density. It is particularly advised whether it is desired to compete with the scientific accelerator in terms of produced electronic beam quality. The second state operates with higher density than the resonance one and it can be more efficient but it requires a series of non linear additional processes among which the auto-focalization and the pulse fragmentation, in general degrading the quality of the outputted electron beam.

The laser active components are supplied and cooled by a power supply and cooling unit 23 (PCA) that is not an object of the present invention, since it is substantially of the conventional type.

The accelerating head 1, receiving the laser pulse through the guiding pipe 3, forms the radiological treatment device according to the present exemplificative embodiment.

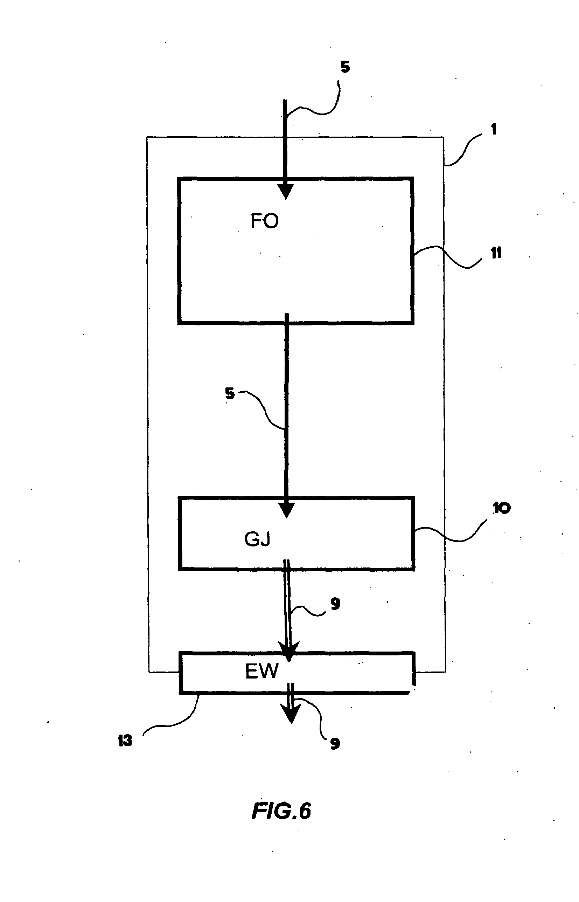

Within the case, the internal structure comprises three optically aligned elements: a focusing optic 11 (FO) of the laser beam 5; a nozzle 10 emitting a pressurized gas jet (GJ) and a window 13 (EW) allowing the output of an electron beam 9 from the head 1, keeping vacuum conditions therein (figure 6).

With this arrangement, a representative case could be sized 40 cm x 25 cm x 25 cm, and weigh about 8 Kg. It can be connected to the guiding pipe 3 by means of an articulated joint such as the aforementioned ones, so as to allow to orientate the device.

The electrons window 13 allows to emit electrons in air towards the surgical site to be treated. It is typically formed by a sheet in a suitable material, thin enough not to interfere with the electrons propagation but capable of standing the pressure difference between inside and outside, about 1 Atmosphere.

As will be explained in the following, these components form a linear accelerator, capable of generating a relativistic electron beam 9, suitable to be used in the IORT technique.

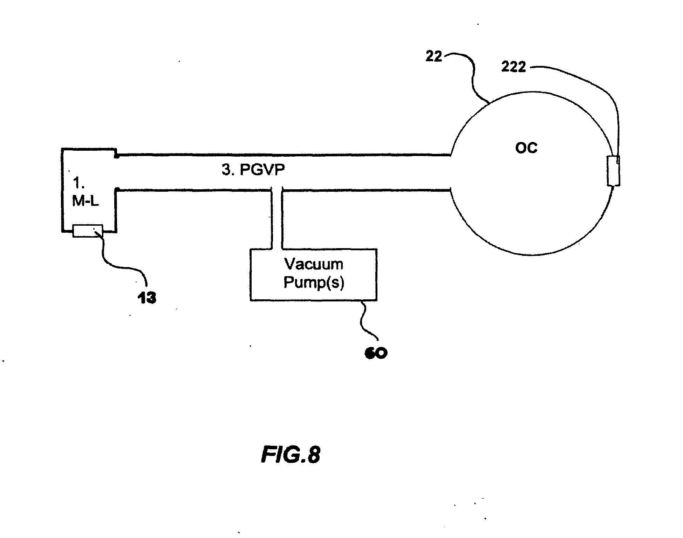

With reference to figure 8, it should be noted that one or more vacuum pumps, shown with the reference numeral 60, can maintain the vacuum conditions required for the optical compressor OC (22), the guiding pipe 3 and the head 1, i.e. the device according to the present exemplificative embodiment. The working conditions of the pump will not be particularly onerous. The laser beam input in the volume under vacuum conditions takes place through the optical window 222, the electrons output from the volume under vacuum conditions through window 13.

The optics of the focusing means can comprise a lens in the case of short pulse regime of operation (duration: 50+200 fs), with a relatively easy and cost effective manufacturing. The lens allows a much easier optical alignment.

In ultra-short pulses regime (duration: < 50 fs), the focusing means advantageously comprise a parabolic mirror, abaxial to the laser propagation axis.

The "F number" of the laser beam focusing optic, i.e. the ratio between the focal length and the non focalized laser beam diameter is comprised between 1.5 and 5.0, advantageously between 2.0 and 4.0. The focalization produces a spot on the gas jet with a diameter comprised between 5 and 20 µm, preferably between 8 and 12 µm, with an intensity at focus comprised between 10¹⁷ and 10²⁰ W/cm², preferably between 10¹⁸ and 10¹⁹W/cm².

The focusing optic is directed to focalize the laser pulses in order to obtain the suitable light intensity required for the pulse incident to the gas jet 50.

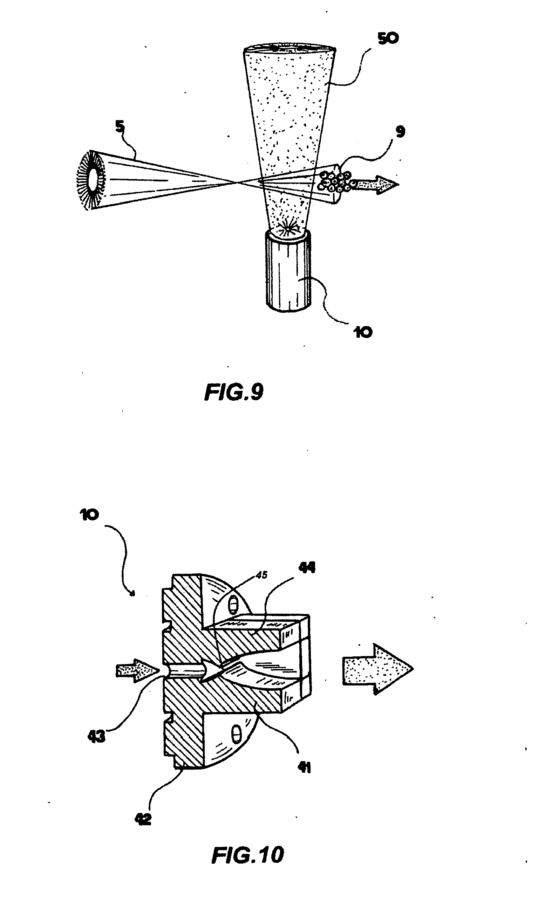

The laser accordingly focalized is capable of providing in the gas jet a gas molecule ionization with a consequent excitation of the plasma wave and pumping of such waves to high amplitudes in a non-linear interaction state. The plasma electrons fraction in the favourable phase is accelerated by the plasma wave supported by laser pulses (figure 9).

The nozzle, shown with 10, is connected to a pressurized tank and is provided with a suitable valve, e.g. a magnetic quick valve, in order to obtain a pulsed gas jet. The output section 41 is provided in a flange 42 through which a conduit 43 is provided (figures 9 and 10). The opening 45 of the output section is preferably rectangular shaped, so as to focalize the laser beam on the greater side allowing a less critical pointing. The nozzle 10 shape is designed so as to obtain an uniform supersonic jet (shock-free), due to a suitable expansion profile 44 interposed between conduit 43 and output section 41. The optimal profile is designed by using fluid mechanics numerical codes.

Moreover, it should be understood that the nozzle hereby described can be comprised in a set of nozzles interchangeable between them, in order to vary the gas jet transversal size and thus also the thickness of the gas jet crossed by the focalized laser beam.

The nozzles output section of the set can be rectangular or circular shaped. A set of nozzles having circular section will comprise a number of nozzles so that the diameter cross-section varies from one nozzle to the other of 0.5 mm in a diameter range from 0.5 mm to 5.0 mm, and of 1.0 mm in the diameter range from 5.0 mm and 10.0 mm.

In case of rectangular nozzle output section, the width of the output section varies in a similar way: from 0.5 mm width to 5.0 mm width of 0.5 mm and from 5.0 mm width to 10,0 mm width of 1.0 mm. Width means the short side of the rectangular section, considering that the laser beam is focalized on the gas jet in correspondence of the long side.

The laser beam is focalized on the gas jet input section border with a longitudinal tolerance comprised between 30 and 150 µm according to the F number of the focusing optic.

The gas speed is comprised between 1.5 and 5.0 Mach, preferably between 2 and 3 Mach. An inert or almost-inert gas choose among a group comprising Helium, Nitrogen, Argon is used. For the present device the use of Argon is preferred, allowing higher relativistic electron currents.

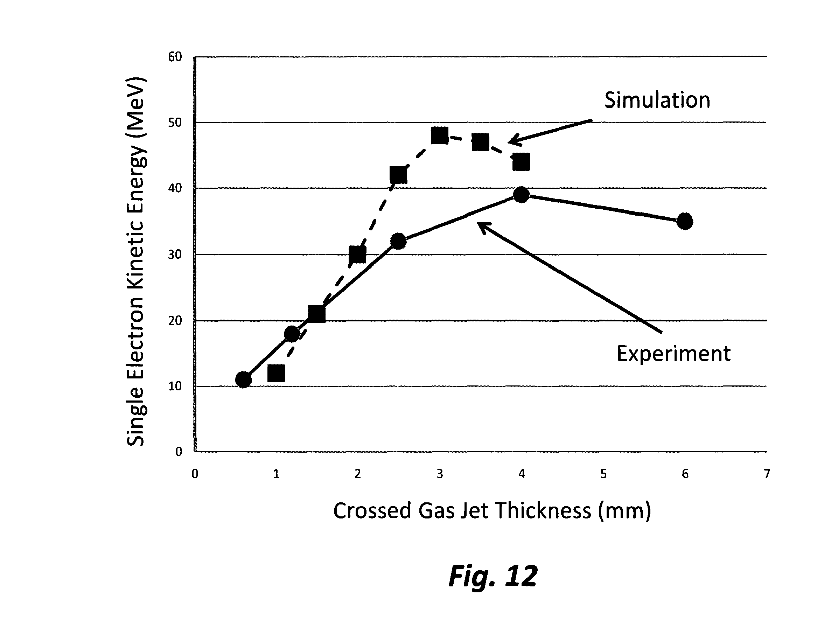

The laser propagation within gas jet and the features of the formed electrons depends on the following parameters:
- thickness *b* crossed by the focalized laser beam by means of the gas jet, defined as the distance between two points wherein the density is equal to a tenth of the maximum density at the jet centre. In the present exemplificative embodiment, this parameter is comprised between 0.5 and 10 mm, preferably between 0.5 and 5 mm. The parameter b is function of the nozzle output section and depends in turn by parameters a and α hereby described. The variation of this parameter allows to vary the kinetic energy of electrons obtained, as shown in the diagram of figure 12 highlighting a monotonic increasing of the energy with thicknesses comprised in the range from 0.6 mm to 4 mm.

- The numerical density (particles number per volume unit, in short density) *nₑ* of free electrons in the plasma produced by laser in the gas. Such density has a maximum in the centre of the nozzle output and longitudinally decreases moving away from the plane and transversally moving away from said hole axis. Densities suitable for the present device are comprised between 10¹⁷ and 10²⁰ el/cm³, preferably between 2x10¹⁸ and 5x10¹⁹ el/cm³. Such density is related to the gas pressure in the tank i.e. at the nozzle inlet 43 and depends in turn by parameters a and α hereby described;
- the distance *a* between laser pulse propagation axis and nozzle output section 41 centre; variations of *a* produce simultaneous variations of *nₑ* (see above) and of *b* (due to jet divergence); and
- angle α between laser propagation axis and nozzle output plane 10; a variation of the density profile of the crossed gas and of *b* will correspond to variations of α.

The front edge (leading edge) of the laser pulse ionizes the gas and produces a plasma with a certain density nₑ of free electrons.

The rest of the pulse excite electronic longitudinal plasma waves, the wavelength λₚ thereof being rigidly connected to the free electrons nₑ density.

In suitable conditions, these plasma waves produces a packet of relativistic kinetic energy electrons, i.e. higher than the rest energy associated to the electron mass (0.5 MeV). A high non linear wave growth speed is reached after few hundreds of µm of laser penetration, and accordingly plasma waves break and then reassemble themselves. In the wave break site, a number of free electrons is caught in phase with the wave acceleration that can accordingly bring their speed until but not over wave phase speed. Therefore, it is not possible to accelerate further, and the limit length for the acceleration is called dephasing length. L_{d}. Then, further limits to the maximum acceleration exist, in particular the focusing length optically determined by the F number and by diffraction, L_{f}, the depletion length Lᵢ after which the laser field decreases under the minimum effective value, the path length L, i.e. the total length of the laser path through the gas jet. The lowest length of the above mentioned four ones will determine the maximum kinetic energy that can be provided to the electrons. Since non-linear processes combined between them are involved, the acceleration effective length can be evaluated only by means of using numerical simulation codes. For the present device, by introducing the involved parameters and by varying them within the range hereby mentioned, acceleration lengths comprised between 0.8 and 5 mm are obtained, the range being narrowed to between 1.2 and 3 mm if the ranges are limited to the preferred values. With 3 mm the kinetic energy of the produced electrons can be over 50 MeV. The total charge generated per pulse remains in the whole range around 1 nC.

It is appropriate for the present device to choose parameters determining an acceleration length of about 3 mm. In this way the kinetic energy could be reduced starting from 50 MeV by reducing the nozzle output section.

Accordingly, it is determined the need of providing the device with adjustment means for adjusting the thickness and the density of the gas jet crossed by the laser beam, in order to optimize the kinetic energy of the electrons obtained as above described. This will be obtained by appropriately varying one or more of the above defined parameters *b, Nₑ,* a and α.

According to the present exemplificative embodiment, said adjustment means operates by varying the distance a of the laser propagation axis from the gas jet output plane, i.e. from the nozzle output section, so as to exploit the gas jet natural divergence.

Moreover, it operates by varying the laser propagation axis tilt α with respect to the nozzle axis, or vice versa. It should be understood that in the present example the two effects hereby described are combined in said adjustment means for adjusting the thickness of the gas jet crossed by the laser propagation axis, but only one of them can be present without departing from the scope of the present invention.

Moreover, such adjustments can be obtained by making nozzles with different opening automatically interchangeable (obtaining discontinuous variations of the electrons kinetic energy) and by making each nozzle mobile, by means of suitable actuators capable of moving it according a linear path corresponding to the output section axis (*a* variation) and rotating it about an axis which is perpendicular to the plane corresponding to the output section axis and to the laser propagation axis

(alpha variation). In the latter two cases continuous variations of the electron beam quality and of the kinetic energy thereof (between two discontinuous values obtained by substituting the nozzle).

As far as it concerns the gas pressure in the tank the useful ranges for the present device are comprised between 5 and 40 Atm, preferably between 10 and 25 Atm.

In the present example, the distance *a* between the propagation axis of the laser pulse and the centre of the nozzle 10 output section 41 can be adjusted between 0.2 and 5.0 mm, preferably between 0.4 and 2.5 mm.

Moreover, the angle α between the laser propagation axis and the nozzle 10 output plane can be adjusted between 0° and 45°, preferably between 12° and 30°.

By means of this adjustment, it is possible to control the energy of the emitted electrons according to the desired penetration in the organic tissues of the surgical site, with energies varying from 1 to 40 MeV and absorbed energy doses in the order of 10 Gy in the point required by the surgeon. In this way, it is certainly possible to obtain a penetration equal to or higher than 15 cm during the IORT procedure.

This dose can be released in a period of time of the order of 1 minute with electronic charges of about 1 nC per pulse, assuming a device repetition frequency of 10 Hz (comparable with the technology used at present). This involves that each application could comprise about 600 laser strokes, assuring a stability within 5% and a measuring uniformity within about 10%.

The above mentioned energy doses, particularly suitable for IORT treatment, are thus obtained by a device that, due to its constituent features, is small sized handy and easy-to-use, easily replaceable, repairable and it does not requires ultra-high-vacuum.

By way of example, in the following some other working parameters are provided in order to define the laser to be used in the above described device:
- Laser wavelength between 300 and 1000 nm; preferably 800 nm (Ti:Sapphire CPA technology).
- Laser pulse peak power between 1 and 30 TW; preferably between 5 and 10 TW.
- Contrast ratio between pulse power and pre-pulse: ≥ 10⁶ - 1 ns before, ≥ 10³ - 2 ps before pulse;
- Laser pulse optical quality factor M² between 1.2 and 3.0
- Main parameters variation from pulse to pulse ≤ 1%

Therefore, the above described invention allows to obtain relevant advantages, broadening the possible application field of IORT techniques.

In particular, the acceleration technique hereby used reduces the acceleration length of a 1000 factor with respect to known art. E.g., in order to produce electrons of 10 MeV, lengths of the order of a millimetre are required instead of a metre ones.

Moreover, it is possible to considerably increase the energy of the incident electrons by modifying the length (jet thickness) of the laser crossed gas, obtaining energies up to even 50 MeV, with thickness variations equal to few millimetres. This can increase the electrons penetration, thus extending IORT technique to the treatment of a wider ranges of tumours.

Furthermore, the use of ultra-high-vacuum conditions (pressures lower than 10⁻⁸ Torr) is not required. This make particularly easy and cost effective the repair and maintenance processes of the whole apparatus.

The electron packets produced according to the known technique based on Radio Frequency have a duration of the order of µs while with the device according to the invention the duration is of the order of ps. Accordingly, the electron current peak is a million times higher, considerably increasing the biological impact and the therapeutic effectiveness of this treatment, but decreasing at the same time any side effect.

It should be understood that the above described device, besides IORT, can be provided in any radiological treatment, even non-medical, requiring the above mentioned energy dose and the above highlighted flexibility and manageability distinctiveness.

As far as it regards the surgical apparatus, it can be manufactured according to a modular arrangement, and the apparatus head can be easily replaced and removed from the operating room, performing outside the operating room any maintenance and repairing operations.

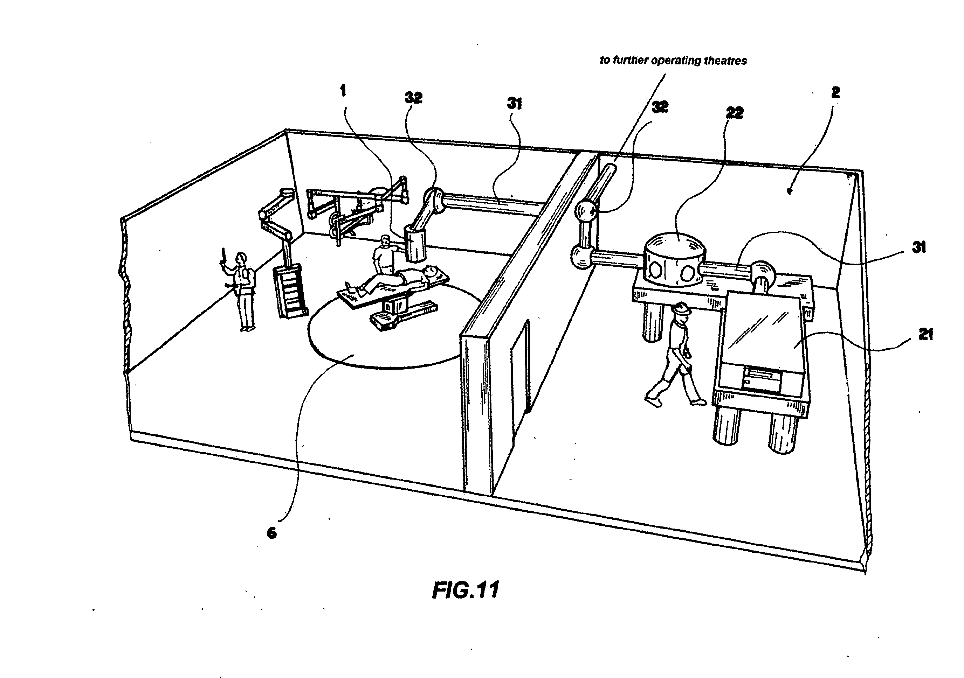

Moreover, the most sensitive and delicate component, i.e. the laser source, is not placed in the operating room and it can be tested, operated and repaired without interfering with the surgical operation. It should be also noted that a single laser can be used for more than one surgical site, even simultaneously (figures 4 and 11). Even the required measures for radio-protecting operators and surgeons are much less strict than in the known art since most of the device (the laser and the laser beam conduit) is not radiogenic and only the mini accelerator requires radio-protection measures. Moreover, from several simulations and measures it appears that the ionizing radiation angular distribution is much more forward concentrated in the case of laser guided mini-accelerators than in the present case of RF cavity.

To the above described radiological treatment device and to the relative surgical apparatus a person skilled in the art, in order to satisfy further and contingent needs, could introduce several further modifications and variants, all however encompassed in the protective scope of the present invention, as defined by the appended claims.

## Claims

1. A radiological treatment device comprising an electron generator having:
• a laser source (2) for generating a laser beam (5);
• a nozzle (10) for vacuum emitting a pressurized gas jet (50); and
• focusing means for focusing (11) said laser beam (5) on said pressurized gas jet (50) nearby the nozzle (10),
**characterized in that** it comprises adjustment means for adjusting the thickness (b) of the gas jet (50) crossed by the laser propagation axis.

2. The device according to claim 1, wherein said adjustment means operates by varying the transversal dimensions of the gas jet.

3. The device according to claim 1 or 2, wherein said adjustment means operates by varying the distance (a) of the laser propagation axis from the gas jet output plane, i.e. from the nozzle (10) output section (41).

4. The device according to any of the preceding claims, wherein said adjustment means operates by varying the laser propagation axis tilt relative to the nozzle axis, or vice versa.

5. The device according to claim 2, wherein the distance (a) between the laser pulse propagation axis and the centre of the nozzle (10) output section (41) is adjusted between 0.2 and 5.0 mm, preferably between 0.4 and 2.5 mm.

6. The device according to claim 3, wherein the angle (alpha) between the laser propagation axis and the nozzle (10) output plane can be adjusted between 0° and 45°, preferably between 12° and 30°.

7. The device according to any of the preceding claims, comprising a case, the internal structure thereof having three substantially optically aligned elements: the focusing means, comprising a focusing optic (11); the nozzle (10) emitting the pressurized gas jet (50) and a window (13) for allowing an electron beam (9) output maintaining a vacuum degree therein.

8. The device according to claim 6, wherein said vacuum degree provides a pressure lower than 0.133 Pa (10⁻³ Torr), preferably lower than 0.013 Pa (10⁻⁴ Torr).

9. The device according to any of the preceding claims, wherein the focusing means optic comprises a lens, the condition of the laser entering the device being of the short pulses type (duration: 100+200 fs)

10. The device according to any of the preceding claims, wherein the focusing means F number, i.e. the ratio between the focal length and the focalized laser incidence point is comprised between 1.5 and 5.0, advantageously between 2.0 and 4.0.

11. The device according to any of the preceding claims, wherein the focalization produces a spot on the gas jet having a diameter comprised between 5 and 20 µm, preferably between 8 and 12 µm, with an intensity at focus comprised between 10¹⁷ and 10²⁰ W/cm², preferably between 10¹⁸ and 10¹⁹ W/cm².

12. The device according to any of the preceding claims, wherein the gas speed from the nozzle (10) is comprised between 1.5 and 5.0 Mach, preferably between 2 and 3 Mach.

13. A surgical apparatus per the radiological treatment, e.g. of the type used for irradiating a surgical site according to the so-called IORT (Intra-Operative Radiological Therapy) technique, comprising at least a device according to any of the preceding claims, wherein the laser source is located in a separated room, the laser being guided by means of one or more tubular guiding pipes (3).

14. The surgical apparatus according to claim 13, wherein the guiding pipe (3) can be oriented and it comprises a plurality of rectilinear tubular elements (31), connected to articulated joints (32) housing an optical element, in particular a mirror (33) mounted on an adjustable support (34) operated by a remote control (4).

15. The surgical apparatus according to claim 13 or 14, wherein the guiding pipe (3) has ordinary vacuum conditions therein, ad a pressure lower than 0.133 Pa (10⁻³ Torr), preferably lower than 0.013 Pa (10⁻⁴ Torr).
